# EUROPEAN PATENT APPLICATION

(11) **EP 0 874 050 A2**
(43) Date of publication of application: **28.10.1998**
(21) Application number: 98300575.2
(22) Date of filing: 27.01.1998
(51) Int. Cl.: C12N 15/15, C07K 14/47, C12N 5/10, C07K 16/18, A61K 31/70, C12Q 1/68, G01N 33/68, A61K 48/00

(54) **Integrin ligand ITGL-TSP**

(30) Priority: 24.04.1997 US 845496
(71) Applicant: SMITHKLINE BEECHAM CORPORATION, Philadelphia Pennsylvania 19103 (US); SMITHKLINE BEECHAM PLC, Brentford, Middlesex TW8 9EP (GB); HUMAN GENOME SCIENCES, INC., Rockville, MD 20850-3338 (US)
(72) Inventor: Jonak, Zdenka L., King of Prussia, Pennsylvania 19406 (US); Trulli, Stephen H., King of Prussia, Pennsylvania 19406 (US); Fronwald, James A., King of Prussia, Pennsylvania 19406 (US); Hastings, Gregg A.,, Rockville, MD 20850 (US); Terrett, Jonathan A., SmithKline Beecham Pharma., Harlow, Essex, CM19 5AW (GB)
(74) Representative: Crump, Julian Richard John

(57) **Abstract**

ITGL-TSP polypeptides and polynucleotides and methods for producing such polypeptides by recombinant techniques are disclosed. Also disclosed are methods for utilizing ITGL-TSP polypeptides and polynucleotides in the design of protocols for the treatment of, angiogenic diseases (cancer, cancer metastasis, chronic inflammatory disorders, rheumatoid arthritis, atherosclerosis, macular degeneration, diabetic retinopathy), restenosis, Alzheimer's disease and tissue remodeling, among others, and diagnostic assays for such conditions.

### Annex to the description

SEQUENCE LISTING

(1) GENERAL INFORMATION

(i) APPLICANTS: SmithKline Beecham Corporation,
SmithKline Beecham p.l.c. and
Human Genome Sciences Inc

(ii) TITLE OF THE INVENTION: Novel Integrin Ligand ITGL-TSP

(iii) NUMBER OF SEQUENCES: 4

(iv) CORRESPONDENCE ADDRESS:
(A) ADDRESSEE: F J Cleveland & Company
(B) STREET: 40/43 Chancery Lane
(C) CITY: London
(D) COUNTY:
(E) COUNTRY: United Kingdom
(F) POST CODE: WC2A 1JQ

(v) COMPUTER READABLE FORM:
(A) MEDIUM TYPE: Diskette
(B) COMPUTER: IBM Compatible
(C) OPERATING SYSTEM: DOS
(D) SOFTWARE: FastSEQ for Windows Version 2.0

(vi) CURRENT APPLICATION DATA:
(A) APPLICATION NUMBER:
(B) FILING DATE: 24-APR-1997
(C) CLASSIFICATION:

(vii) PRIOR APPLICATION DATA:
(A) APPLICATION NUMBER:
(B) FILING DATE:

(viii) ATTORNEY/AGENT INFORMATION:
(A) NAME: CRUMP, Julian Richard John
(B) GENERAL AUTHORISATION NUMBER: 37127 and 37129
(C) REFERENCE/DOCKET NUMBER: GH-70000

(ix) TELECOMMUNICATION INFORMATION:

(A) TELEPHONE: +44 171 405 5875
(B) TELEFAX: +44 171 831 0749
(C) TELEX:

(2) INFORMATION FOR SEQ ID NO: 1:

(i) SEQUENCE CHARACTERISTICS:
(A) LENGTH: 4014 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 1:

CCCACGCGTC CGCCCACGCG TCCGGCGGCT CCGAGCCAGG GGCTATTGCA AAGCCAGGGT 60
GCGCTACCGG ACGGAGAGGG GAGAGCCCTG AGCAGAGTGA GCAACATCGC AGCCAAGGCG 120
GAGGCCGAAG AGGGGCGCCA GGCACCAATC TCCGCGTTGC CTCAGCCCCG GAGGCGCCCC 180
AGAGCGCTTC TTGTCCCAGC AGAGCCACTC TGCCTGCGCC TGCCTCTCAG TGTCTCCAAC 240
TTTGCGCTGG AAGAAAAACT TCCCGCGCGC CGGCAGAACT GCAGCGCCTC CTCTTAGTGA 300
CTCCGGGAGC TTCGGCTGTA GCCGGCTCTG CGCGCCCTTC CAACGAATAA TAGAAATTGT 360
TAATTTTAAC AATCCAGAGC AGGCCAACGA GGCTTTGCTC TCCCGACCCG AACTAAAGCT 420
CCCTCGCTCC GTGCGCTGCT ACGAGCGGTG TCTCCTGGGG CTCCAATGCA GCGAGCTGTG 480
CCCGAGGGGT TCGGAAGGCG CAAGCTGGGC AGCGACATGG GGAACGCGGA GCGGGCTCCG 540
GGGTCTCGGA GCTTTGGGCC CGTACCCACG CTGCTGCTGC TCGCCGCGGC GCTACTGGCC 600
GTGTCGGACG CACTCGGGCG CCCCTCCGAG GAGGACGAGG AGCTAGTGGT GCCGGAGCTG 660
GAG[?]G[?]G[?]CC CGGGACACGG GACCACGCGC CTCCGCCTGC ACGCCTTTGA CCAGCAGCTG 720
G[?]TCTGGAC[?] TGCC[?]CCCGA CAGCAGCTTT TTGGCGCCCG GCTT[?]ACGCT CCAGAACGTG 780
GGG[?]CGCAAAT CCGGCT[?]CGA CACC[?]CGCTT [?]CGGAAACCG ACCTGGCGCA CTGCTTCTAC 840
TCCGGCACCG TGAATGGCGA TCCCAGCTCG GCTGCCGCCC TCAGCCTCTG CGAGGGCGTG 900
CGCGGCGCCT TCTACCTGCT GGGGGAGGCG TATTTCATCC AGCCGCTGCC CGCCGCCAGC 960
GAGCGCCTCG CCACCGCCGC CCCAGGGGAG AAGCCGCCGG CACCACTACA GTTCCACCTC 1020
CTGCGGCGGA ATCGGCAGGG CGACGTAGGC GGCACGTGCG GGGTCGTGGA CGACGAGCCC 1080
CGGCCGACTG GGAAAGCGGA GACCGAAGAC GAGGACGAAG GGACTGAGGG CGAGGACGAA 1140
GGGCCTCAGT GGTCGCCGCA GGACCCGGCA CTGCAAGGCG TAGGACAGCC CACAGGAACT 1200
GGAAGCATAA GAAAGAAGCG ATTTGTGTCC AGTCACCGCT ATGTGGAAAC CATGCTTGTG 1260
GCAGACCAGT CGATGGCAGA ATTCCACGGC AGTGGTCTAA AGCATTACCT TCTCACGTTG 1320
TTTTCGGTGG CAGCCAGATT GTACAAACAC CCCAGCATTC GTAATTCAGT TAGCCTGGTG 1380
GTGGTGAAGA TCTTGGTCAT CCACGATGAA CAGAAGGGGC CGGAAGTGAC CTCCAATGCT 1440
GCCCTCACTC TGCGGAACTT TTGCAACTGG CAGAAGCAGC ACAACCCACC CAGTGACCGG 1500
GATGCAGAGC ACTATGACAC AGCAATTCTT TTCACCAGAC AGGACTTGTG TGGGTCCCAG 1560

ACATGTGATA CTCTTGGGAT GGCTGATGTT GGAACTGTGT GTGATCCGAG CAGAAGCTGC 1620
TCCGTCATAG AAGATGATGG TTTACAAGCT GCCTTCACCA CAGCCCATGA ATTAGGCCAC 1680
GTGTTTAACA TGCCACATGA TGATGCAAAG CAGTGTGCCA GCCTTAATGG TGTGAACCAG 1740
GATTCCCACA TGATGGCGTC AATGCTTTCC AACCTGGACC ACAGCCAGCC TTGGTCTCCT 1800
TGCAGTGCCT ACATGATTAC ATCATTTCTG GATAATGGTC ATGGGGAATG TTTGATGGAC 1860
AAGCCTCAGA ATCCCATACA GCTCCCAGGC GATCTCCCTG GCACCTCGTA CGATGCCAAC 1920
CGGCAGTGCC AGTTTACATT TGGGGAGGAC TCCAAACACT GCCCTGATGC AGCCAGCACA 1980
TGTAGCACCT TGTGGTGTAC CGGCACCTCT GGTGGGGTGC TGGTGTGTCA AACCAAACAC 2040
TTCCCGTGGG CGGATGGCAC CAGCTGTGGA GAAGGGAAAT GGTGTATCAA CGGCAAGTGT 2100
GTGAACAAAA CCGACAGAAA GCATTTTGAT ACGCCTTTTC ATGGAAGCTG GGGAATGTGG 2160
GGGCCTTGGG GAGACTGTTC GAGAACGTGC GGTGGAGGAG TCCAGTACAC GATGAGGGAA 2220
TGTGACAACC CAGTCCCAAA GAATGGAGGG AAGTACTGTG AAGGCAAACG AGTGCGCTAC 2280
AGATCCTGTA ACCTTGAGGA CTGTCCAGAC AATAATGGAA AAACCTTTAG AGAGGAACAA 2340
TGTGAAGCAC ACAACGAGTT TTCAAAAGCT TCCTTTGGGA GTGGGCCTGC GGTGGAATGG 2400
ATTCCCAAGT ACGCTGGCGT CTCACCAAAG GACAGGTGCA AGCTCATCTG CCAAGCCAAA 2460
GGCATTGGCT ACTTCTTCGT TTTGCAGCCC AAGGTTGTAG ATGGTACTCC ATGTAGCCCA 2520
GATTCCACCT CTGTCTGTGT GCAAGGACAG TGTGTAAAAG CTGGTTGTGA TCGCATCATA 2580
GACTCCAAAA AGAAGTTTGA TAAATGTGGT GTTTGCGGGG GAAATGGATC TACTTGTAAA 2640
AAAATATCAG GATCAGTTAC TAGTGCAAAA CCTGGATATC ATGATATCAT CACAATTCCA 2700
ACTGGAGCCA CCAACATCGA AGTGAAACAG CGGAACCAGA GGGGATCCAG GAACAATGGC 2760
AGCTTTCTTG CCATCAAAGC TGCTGATGGC ACATATATTC TTAATGGTGA CTACACTTTG 2820
TCCACCTTAG AGCAAGACAT TATGTACAAA GGTGTTGTCT TGAGGTACAG CGGCTCCTCT 2880
GCGGCATTGG AAAGAATTCG CAGCTTTAGC CCTCTCAAAG AGCCCTTGAC CATCCAGGTT 2940
CTTACTGTGG GCAATGCCCT TCGACCTAAA ATTAAATACA CCTACTTCGT AAAGAAGAAG 3000
AAGGAATCTT TCAATGCTAT CCCCACTTTT TCAGCATGGG TCATTGAAGA GTGGGGCGAA 3060
TGTTCTAAGT CATGTGAATT GGGTTGGCAG AGAAGACTGG TAGAATGCCG AGACATTAAT 3120
GGACAGCCTG CTTCCGAGTG TGCAAAGGAA GTGAAGCCAG CCAGCACCAG ACCTTGTGCA 3180
GACCATCCCT GCCCCCAGTG GCAGCTGGGG GAGTGGTCAT CATGTTCTAA GACCTGTGGG 3240
A[?]GCCTTACA AAAAAACAAG CTTGAAGTCT CTGTCCCATG ATGGAGGGGT GTTATCTCAT 3300
GACAGC[?][?]TG A[?]CCT[?]TAAA GAAACCTAAA CATTTCATAG A[?]TTTTGCAC AATGGCAGAA [?][?][?][?]
TGCAGTTAAG TGGTTTAAGT GGTGTTAGCT TTGAGGGCAA GGCAAAGTGA GGAAGGGCTG 3420
GTGCAGGGAA AGCAAGAAGG CTGGAGGGAT CCAGCGTATC TTGCCAGTAA CCAGTGAGGT 3480
GTATCAGTAA GGTGGGATTA TGGGGGTAGA TAGAAAAGGA GTTGAATCAT CAGAGTAAAC 3540
TGCCAGTTGC AAATTTGATA GGATAGTTAG TGAGGATTAT TAACCTCTGA GCAGTGATAT 3600
AGCATAATAA AGCCCCGGGC ATTATTATTA TTATTTCTTT TGTTACATCT ATTACAAGTT 3660
TAGAAAAAAC AAAGCAATTG TCAAAAAAAG TTAGAACTAT TACAACCCCT GTTTCCTGGT 3720
ACTTATCAAA TACTTAGTAT CATGGGGGTT GGGAAATGAA AAGTAGGAGA AAAGTGAGAT 3780
TTTACTAAGA CCTGTTTTAC TTTACCTCAC TAAACAATGG GGGGAGAAAG GAGTACAAAT 3840
AGGATCTTTT GACCAGCACT GTTTATGGGC TGCTATGGTT TCAGAGAATG TTTATACATT 3900
ATTTCTACCG AGGATTTAAA ACTTCCAGAT TGTTCCAACA TGGAGAGGAA AGGCTCAGGC 3960
AACGTGGAAA TAACGCAATG GGCTTCCCCC TTCCCTTTTT GGGACCCACT CCAG 4014

(2) INFORMATION FOR SEQ ID NO: 2:

(i) SEQUENCE CHARACTERISTICS:
(A) LENGTH: 967 amino acids
(B) TYPE: amino acid
(C) STRANDEDNESS: single
(D) TOPOLOGY: linear
(ii) MOLECULE TYPE: protein

(xi) SEQUENCE DESCRIPTION: SEQ ID NO: 2:

(2) INFORMATION FOR SEQ ID NO: 3:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: cDNA
(xi) SEQUENCE DESCRIPTION: SEQ ID NO: 3:

ACTGTGTGTG ATCCGAG                                                    17

(2) INFORMATION FOR SEQ ID NO: 4:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 4:

GTTGGAAAGC ATTGACG                                                    17

## Description

### FIELD OF INVENTION

This invention relates to newly identified polynucleotides, polypeptides encoded by them and to the use of such polynucleotides and polypeptides, and to their production. More particularly, the polynucleotides and polypeptides of the present invention relate to thrombospondin-metalloproteinase family, hereinafter referred to as ITGL-TSP. The invention also relates to inhibiting or activating the action of such polynucleotides and polypeptides.

### BACKGROUND OF THE INVENTION

ITGL-TSP is a novel thrombospondin (metalloproteinase)-like gene which could have multifunctional activity in normal and disease states. The homology to the thrombospondin type 1 (TSP-1) would "predict" that ITGL-TSP could have similar functions such as TSP-1. TSP-1 modulates aggregation of platelets, formation and lysis of fibrin, adhesion and migration of cells and progression of cells through the growth cycle. TSP-1 is implicated as a potential regulator of tumor growth and metastasis. Conflicting observations suggest that overexpression of TSP-1 causes "increased or suppressed" tumor growth. TSP-1 is a homotrimer with different functional domains, some of which serve as receptor recognizing regions. One of the important functions has been its ability to bind to integrins, such as aVb3, aIIbb3 and other unknown integrin receptors. Integrins are a large family of cell surface receptors tnat mediate cell to cell as well as cell to matrix adhesion. Structurally, integrins consist of a heterodimer of an a and b chain. Each subunit has a large N-terminal extracellular domain followed by a transmembrane domain and a short C-terminal cytoplasmic region. Some receptors share a common b chain while having different a chains. ITGL-TSP could be a such novel ligand which could play an important role in different diseases.

The role of ITGL-TSP as an integrin ligand is of great interest due to its potential function in angiogenesis. Numerous angiogenic-related disorders have been described and the role of TSP-1 has been claimed in cancer/cancer metastasis. Our own research indicates that ITGL-TSP is "expressed" in numerous tissues (e.g., ovary, aorta, heart, prostate, placenta, skeletal muscle ...). From our data we estimated that ITGL-TSP gene maps to human chromosome 21q21. This is a similar chromosomal location to amyloid precursor protein (APP), enterokinases (enzymes that activates trypsinogen by converting it to trypsin) and genes responsible for Alzheimer's disease. The homology of the ITGL-TSP to the hemorrhagic toxin/metalloproteases would assign to the ITGL-TSP proteolytic functions (proteolyze extracellular matrix or basement membrane proteins). In summary, the role of ITGL-TSP as a ligand to the integrin receptors with metalloprotease activity fits its assigned role in angiogenesis, Alzheimers disease and tissue remodeling. This indicates that the thrombospondin-metalloproteinase family has an established, proven history as therapeutic targets. Clearly there is a need for identification and characterization of further members of the thrombospondin-metalloproteinase family which can play a role in preventing, ameliorating or correcting dysfunctions or diseases, including, but not limited to, angiogenic diseases (cancer, cancer metastasis, chronic inflammatory disorders, rheumatoid arthritis, atherosclerosis, macular degeneration, diabetic retinopathy), restenosis, Alzheimer's disease and tissue remodeling.

### SUMMARY OF THE INVENTION

In one aspect, the invention relates to ITGL-TSP polypeptides and recombinant materials and methods for their production. Another aspect of the invention relates to methods for using such ITGL-TSP polypeptides and polynucleotides. Sucn uses include the treatment of angiogenic diseases (cancer, cancer metastasis, chronic inflammatory disorders, rheumatoid arthritis, atherosclerosis, macular degeneration, diabetic retinopathy), restenosis, Alzheimer's disease and tissue remodeling, among others. In still another aspect, the invention relates to methods to identify agonists and antagonists using the materials provided by the invention, and treating conditions associated with ITGL-TSP imbalance with the identified compounds. Yet another aspect of the invention relates to diagnostic assays for detecting diseases associated with inappropriate ITGL-TSP activity or levels.

### DESCRIPTION OF THE INVENTION

### Definitions

The following definitions are provided to facilitate understanding of certain terms used frequently herein.

"ITGL-TSP" refers, among others, generally to a polypeptide having the amino acid sequence set forth in SEQ ID NO:2 or an allelic variant thereof.

"ITGL-TSP activity or ITGL-TSP polypeptide activity" or "biological activity of the ITGL-TSP or ITGL-TSP polypeptide" refers to the metabolic or physiologic function of said ITGL-TSP including similar activities or improved activities or these activities with decreased undesirable side-effects. Also included are antigenic and immunogenic activities of said ITGL-TSP.

"ITGL-TSP gene" refers to a polynucleotide having the nucleotide sequence set forth in SEQ ID NO:1 or allelic variants thereof and/or their complements.

"Antibodies" as used herein includes polyclonal and monoclonal antibodies, chimeric, single chain, and humanized antibodies, as well as Fab fragments, including the products of an Fab or other immunoglobulin expression library.

"Isolated" means altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein.

"Polynucleotide" generally refers to any polyribonucleotide or polydeoxribonucleotide, which may be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces . relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide Also, a given polypeptide may contain many types of modifications. Polypepitdes may be branched as a result of ubiquitination, and they may be cyclic, with or without branching. Cyclic, branched and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, PROTEINS - STRUCTURE AND MOLECULAR PROPERTIES, 2nd Ed., T. E. Creighton, W. H. Freeman and Company, New York, 1993 and Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York, 1983; Seifter *et al.,* "Analysis for protein modifications and nonprotein cofactors", *Meth Enzymol* (1990) 182:626-646 and Rattan *et al.,* "Protein Synthesis: Posttranslational Modifications and Aging", *Ann NY Acad Sci* (1992) 663:48-62.

"Variant" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitution, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

"Identity" is a measure of the identity of nucleotide sequences or amino acid sequences. In general, the sequences are aligned so that the highest order match is obtained. "Identity *per se* has an art-recognized meaning and can be calculated using published techniques. See, e.g.: (COMPUTATIONAL MOLECULAR BIOLOGY, Lesk, A.M., ed., Oxford University Press, New York, 1988; BIOCOMPUTING: INFORMATICS AND GENOME PROJECTS, Smith, D.W., ed., Academic Press, New York, 1993; COMPUTER ANALYSIS OF SEQUENCE DATA, PART I, Griffin, A.M., and Griffin, H.G., eds., Humana Press, New Jersey, 1994; SEQUENCE ANALYSIS IN MOLECULAR BIOLOGY, von Heinje, G., Academic Press, 1987; and SEQUENCE ANALYSIS PRIMER, Gribskov, M. and Devereux, J., eds., M Stockton Press, New York, 1991). While there exist a number of methods to measure identity between two polynucleotide or polypeptide sequences, the term "identity" is well known to skilled artisans (Carillo, H., and Lipton, D., *SIAM J Applied Math* (1988) 48:1073). Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., *SIAM J Applied Math* (1988) 48:1073. Methods to determine identity and similarity are codified in computer programs. Preferred computer program methods to determine identity and similarity between two sequences include, but are not limited to, GCS program package (Devereux, J., *et al., Nucleic Acids Research* (1984) 12(1):387), BLASTP, BLASTN, FASTA (Atschul, S.F. *et al., J Molec Biol* (1990) 215:403).

As an illustration, by a polynucleotide having a nucleotide sequence having at least, for example, 95% "identity" to a reference nucleotide sequence of SEQ ID NO: 1 is intended that the nucleotide sequence of the polynucleotide is identical to the reference sequence except that the polynucleotide sequence may include up to five point mutations per each 100 nucleotides of the reference nucleotide sequence of SEQ ID NO: 1. In other words, to obtain a polynucleotide having a nucleotide sequence at least 95 % identical to a reference nucleotide sequence, up to 5 % of the nucleotides in the reference sequence may be deleted or substituted with another nucleotide, or a number of nucleotides up to 5 % of the total nucleotides in the reference sequence may be inserted into the reference sequence. These mutations of the reference sequence may occur at the 5 or 3 terminal positions of the reference nucleotide sequence or anywhere between those terminal positions, interspersed either individually among nucleotides in the reference sequence or in one or more contiguous groups within the reference sequence.

Similarly, by a polypeptide having an amino acid sequence having at least, for example, 95 % "identity" to a reference amino acid sequence of SEQ ID NO:2 is intended that the amino acid sequence of the polypeptide is identical to the reference sequence except that the polypeptide sequence may include up to five amino acid alterations per each 100 amino acids of the reference amino acid of SEQ ID NO: 2. .In other words, to obtain a polypeptide having an amino acid sequence at least 95% identical to a reference amino acid sequence, up to 5 % of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

### Polypeptides of the Invention

In one aspect, the present invention relates to ITGL-TSP polypeptides. The ITGL-TSP polypeptides include the polypeptide of SEQ ID NO:2; as well as polypeptides comprising the amino acid sequence of SEQ ID NO: 2; and polypeptides comprising the amino acid sequence which have at least 80% identity to that of SEQ ID NO:2 over its entire length, and still more preferably at least 90% identity, and even still more preferably at least 95 % identity to SEQ ID NO: 2. Furthermore, those with at least 97-99% are highly preferred. Also included within ITGL-TSP polypeptides are polypeptides having the amino acid sequence which have at least 80% identity to the polypeptide having the amino acid sequence of SEQ ID NO:2 over its entire length, and still more preferably at least 90% identity, and still more preferably at least 95 % identity to SEQ ID NO:2. Furthermore, those with at least 97-99% are highly preferred. Preferably ITGL-TSP polypeptide exhibit at least one biological activity of ITGL-TSP.

The ITGL-TSP polypeptides may be in the form of the "mature" protein or may be a part of a larger protein such as a fusion protein. It is often advantageous to include an additional amino acid sequence which contains secretory or leader sequences, pro-sequences, sequences which aid in purification such as multiple histidine residues, or an additional sequence for stability during recombinant production.

Fragments of the ITGL-TSP polypeptides are also included in the invention. A fragment is a polypeptide having an amino acid sequence that entirely is the same as part, but not all, of the amino acid sequence of the aforementioned ITGL-TSP polypeptides. As with ITGL-TSP polypeptides, fragments may be "free-standing," or comprised within a larger polypeptide of which they form a part or region, most preferably as a single continuous region. Representative examples of polypeptide fragments of the invention, include, for example, fragments from about amino acid number 1-20, 2140, 41-60, 61-80, 81-100, and 101 to the end of the ITGL-TSP polypeptide. In this context "about" includes the particularly recited ranges larger or smaller by several, 5, 4, 3, 2 or 1 amino acid at either extreme or at both extremes.

Preferred fragments include, for example, truncation polypeptides having the amino acid sequence of ITGL-TSP polypeptides, except for deletion of a continuous series of residues that includes the amino terminus, or a continuous series of residues that includes the carboxyl terminus or deletion of two continuous series of residues, one including the amino terminus and one including the carboxyl terminus. Also preferred are fragments characterized by structural of functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments. Biologically active fragments are those that mediate ITGL-TSP activity, including those with a similar activity or an improved activity, or with a decreased undesirable activity. Also included are those that are antigenic or immunogenic in an animal, especially in a human.

Preferably, all of these polypeptide fragments retain the biological activity of the ITGL-TSP, including antigenic activity. Variants of the defined sequence and fragments also form part of the present invention. Preferred variants are those that vary from the referents by conservative amino acid substitutions -- i.e., those that substitute a residue with another of like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr. Particularly preferred are variants in which several, 5-10, 1-5, or 1-2 amino acids are substituted, deleted, or added in any combination.

The ITGL-TSP polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

### Polynucleotides of the Invention

Another aspect of the invention relates to ITGL-TSP polynucleotides. ITGL-TSP polynucleotides include isolated polynucleotides which encode the ITGL-TSP polypeptides and fragments, and polynucleotides closely related thereto. More specifically, ITGL-TSP polynucleotide of the invention include a polynucleotide comprising the nucleotide sequence set forth in SEQ ID NO: 1 encoding a ITGL-TSP polypeptide of SEQ ID NO: 2, and polynucleotide having the particular sequence of SEQ ID NO:1. ITGL-TSP polynucleotides further include a polynucleotide comprising a nucleotide sequence that has at least 80% identity to a nucleotide sequence encoding the ITGL-TSP polypeptide of SEQ ID NO:2 over its entire length, and a polynucleotide that is at least 80% identical to that having SEQ ID NO:1 over its entire length. In this regard, polynucleotides at least 90% identical are particularly preferred, and those with at least 95 % are especially preferred. Furthermore, those with at least 97% are highly preferred and those with at least 98-99% are most highly preferred, with at least 99% heing the most preferred. Also included under ITGL-TSP polynucleotides are a nucleotide sequence which has sufficient identity to a nucleotide sequence contained in SEQ ID NO:1 to hybridize under conditions useable for amplification or for use as a probe or marker. The invention also provides polynucleotides which are complementary to such ITGL-TSP polynucleotides.

The ITGL-TSP of the invention is structurally related to other proteins of the thrombospondin-metalloproteinase family, as shown by the results of sequencing the cDNA encoding human ITGL-TSP. The cDNA sequence contains an open reading frame encoding a polypeptide of 967 amino acids. The amino acid sequence of Table 1 (SEQ ID NO:2) has about 100% identity in 63 amino acid residues and 95% identity in 721 . amino acid residues (using Bestfit GCG) with mouse ADAM (A cellular disintegrin and metalloproteinase) (K.Kuno et al., JBC 272: 556-562, 1997). The sequence also has some homology to the human thrombospondin-1 (S. C. Hsu et al., Cancer Research 56: 5684-5691, 1996). The nucleotide sequence of Table 1 (SEQ ID NO:1) has about 82% identity (over the coding region) and 79% identity (over the entire sequence) (using Bestfit, GCG ) in 2370 (coding) and 3129 (entire sequence) nucleotide residues with ADAM mouse gene (K.Kuno et al., JBC 272:556-562, 1997).

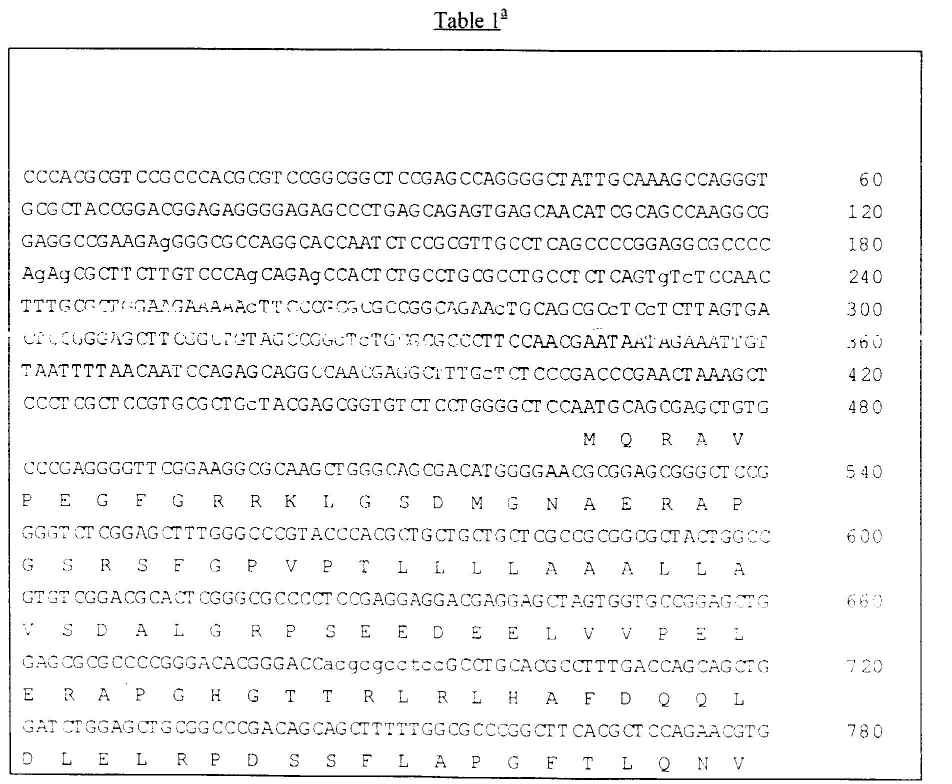

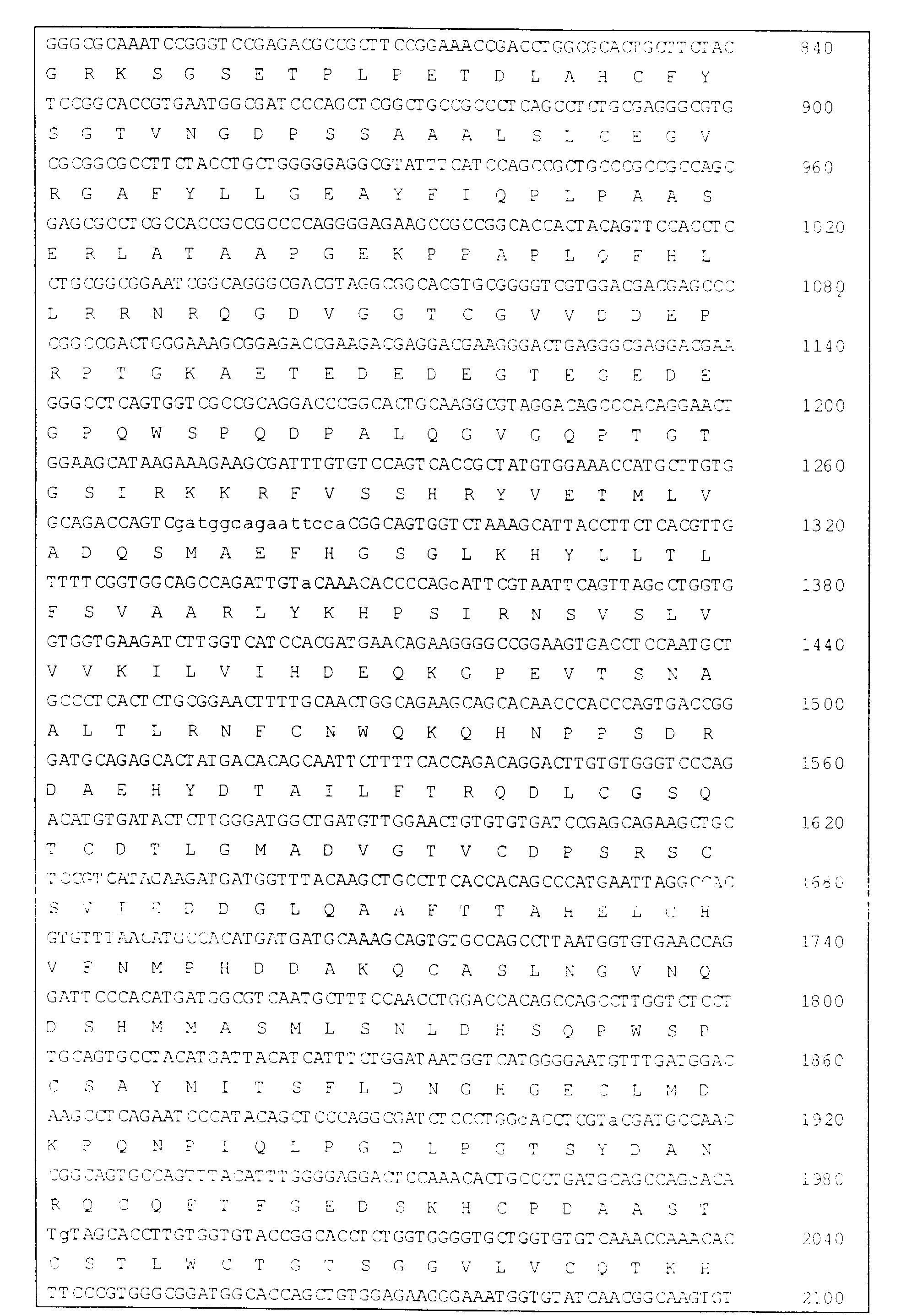

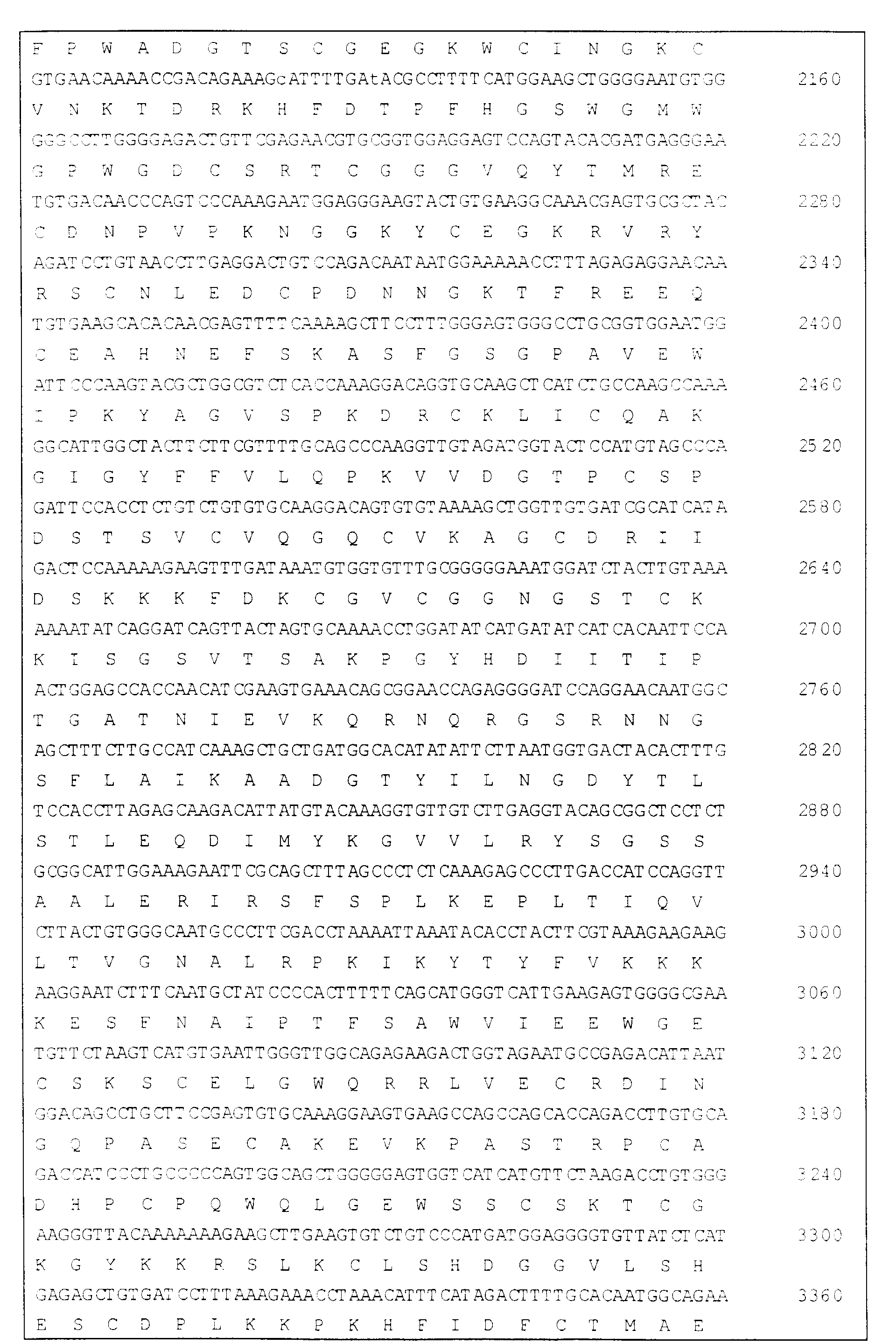

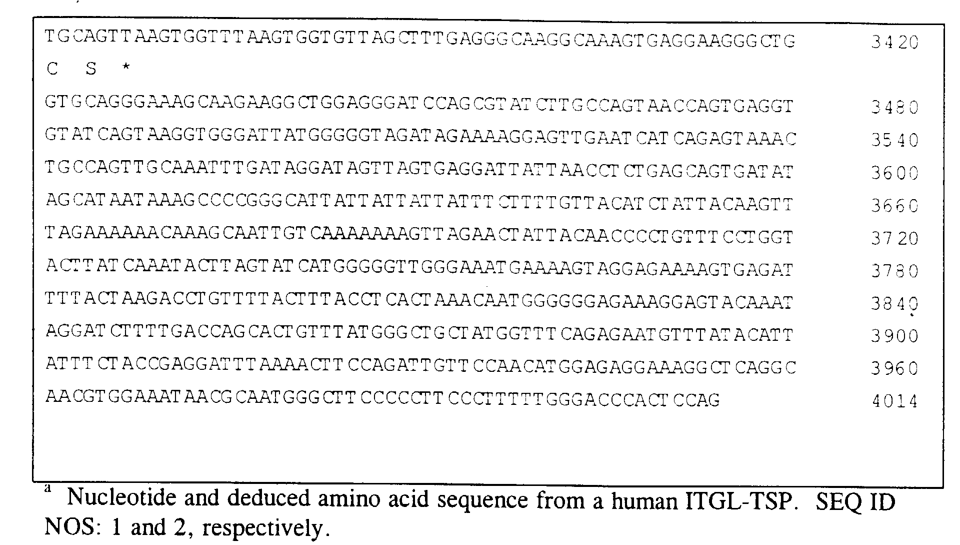

One polynucleotide of the present invention encoding ITGL-TSP may be obtained using standard cloning and screening, from a cDNA library derived from mRNA in cells of human adipocytes using the expressed sequence tag (EST) analysis (Adams, M.D., *et al. Science* (1991) 252:1651-1656; Adams, M.D. *et al., Nature,* (1992) *355:632-634;* Adams, M.D., *et al., Nature* (1995) 377 Supp:3-174). Polynucleotides of the invention can also be obtained from natural sources such as genomic DNA libraries or can be synthesized using well known and commercially available techniques.

The nucleotide sequence encoding the ITGL-TSP polypeptide of SEQ ID NO:2 may be identical to the polypeptide encoding sequence contained in Table 1 (SEQ ID NO: 1), or it may be a sequence, which as a result of the redundancy (degeneracy) of the genetic code, also encodes the polypeptide of SEQ ID NO:2.

When the polynucleotides of the invention are used for the recombinant production of the ITGL-TSP polypeptide, the polynucleotide may include the coding sequence for the mature polypeptide or a fragment thereof, by itself; the coding sequence for the mature polypeptide or fragment in reading frame with other coding sequences, such as those encoding a leader or secretory sequence, a pre-, or pro- or prepro- protein Sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) and described in Gentz *et al., Proc Natl Acad Sci USA* (1989) 86:821-824, or is an HA tag. The polynucleotide may also contain non-coding 5' and 3' sequences, such as transcribed, non-translated sequences, splicing and polyadenylation signals, ribosome binding sites and sequences that stabilize mRNA.

Further preferred embodiments are polynucleotides encoding ITGL-TSP variants comprise the amino acid sequence ITGL-TSP polypeptide of Table 1 (SEQ ID NO:2) in which several, 5-10, 1-5, 1-3, 1-2 or 1 amino acid residues are substituted, deleted or added, in any combination.

The present invention further relates to polynucleotides that hybridize to the herein above-described sequences. In this regard, the present invention especially relates to polynucleotides which hybridize under stringent conditions to the herein above-described polynucleotides. As herein used, the term "stringent conditions" means hybridization will occur only if there is at least 95 % and preferably at least 97 % identity between the sequences.

Polynucleotides of the invention, which are identical or sufficiently identical to a nucleotide sequence contained in SEQ ID NO:1 or a fragment thereof, may be used as hybridization probes for cDNA and genomic DNA, to isolate full-length cDNAs and genomic clones encoding the ITGL-TSP polypeptide and to isolate cDNA and genomic clones of other genes that have a high sequence similarity to the ITGL-TSP gene. Such hybridization techniques are known to those of skill in the art. Typically these nucleotide sequences are 80% identical, preferably 90% identical, more preferably 95% identical to that of the referent. The probes generally will comprise at least 15 nucleotides. Preferably, such probes will have at least 30 nucleotides and may have at least 50 nucleotides. Particularly preferred probes will range between 30 and 50 nucleotides.

In one embodiment, to obtain a polynucleotide encoding ITGL-TSP polypeptide comprises the steps of screening an appropriate library under stingent hybridization conditions with a labeled probe having the SEQ ID NO: 1 or a fragment thereof; and isolating full-length cDNA and genomic clones containing said polynucleotide sequence. Such hybridization techniques are well known to those of skill in the art. Stringent hybridization conditions are as defined above or alternatively conditions under overnight incubation at 42°C in a solution comprising: 50% formamide, 5xSSC (150mM NaCl, 15mM trisodium citrate), 50 mM sodium phosphate (pH7.6), 5x Denhardt's solution, 10 % dextran sulfate, and 20 microgram/ml denatured, sheared salmon sperm DNA, followed by washing the filters in 0.1x SSC at about 65°C.

The polynucleotides and polypeptides of the present invention may be employed as research reagents and materials for discovery of treatments and diagnostics to animal and human disease.

### Vectors, Host Cells, Expression

The present invention also relates to vectors which comprise a polynucleotide or polynucleotides of the present invention, and host cells which are genetically engineered with vectors of the invention and to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Introduction of polynucleotides into host cells can be effected by methods described in many standard laboratory manuals, such as Davis et al., *BASIC METHODS IN MOLECULAR BIOLOGY* (1986) and Sambrook et al., *MOLECULAR CLONING: A LABORATORY MANUAL*, 2nd Ed., Cold Spring Harbor Laboratory Press, Cold Spring Harbor, N.Y. 1989) such as calcium phosphate transfection, DEAE-dextran mediated transfection, transvection, microinjection, cationic lipid-mediated transfection, electroporation, transduction, scrape loading, ballistic introduction or infection.

Representative examples of appropriate hosts include bacterial cells, such as streptococci, staphylococci, *E. coli, Streptomyces* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used. Such systems include, among others, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression. Generally, any system or vector suitable to maintain, propagate or express polynucleotides to produce a polypeptide in a host may be used. The appropriate nucleotide sequence may be inserted into an expression system by any of a variety of well-known and routine techniques, such as, for example, those set forth in Sambrook *et al., MOLECULAR CLONING, A LABORATORY MANUAL* (*supra*).

For secretion of the translated protein into the lumen of the endoplasmic reticulum, into the periplasmic space or into the extracellular environment, appropriate secretion signals may be incorporated into the desired polypeptide. These signals may be endogenous to the polypeptide or they may be heterologous signals.

If the ITGL-TSP polypeptide is to be expressed for use in screening assays, generally, it is preferred that the polypeptide be produced at the surface of the cell. In this event, the cells may be harvested prior to use in the screening assay. If the ITGL-TSP polypeptide is secreted into the medium, the medium can be recovered in order to recover and purify the polypeptide; if produced intracellularly, the cells must first be lysed before the polypeptide is recovered.
ITGL-TSP polypeptides can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during isolation and or purification.

### Diagnostic Assays

This invention also relates to the use of ITGL-TSP polynucleotides for use as diagnostic reagents. Detection of a mutated form of the ITGL-TSP gene associated with a dysfunction will provide a diagnostic tool that can add to or define a diagnosis of a disease or susceptibility to a disease which results from under-expression, overexpression or altered expression of ITGL-TSP. Individuals carrying mutations in the ITGL-TSP gene may be detected at the DNA level by a variety of techniques.

Nucleic acids for diagnosis may be obtained from a subject's cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled ITGL-TSP nucleotide sequences. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing. See, e.g., Myers *et al., Science* (1985) 230:1242. Sequence changes at specific locations may also be revealed by nuclease protection assays, such as RNase and S1 protection or the chemical cleavage method. See Cotton *et al., Proc Natl Acad Sci USA* (1985) 85: 4397-4401. In another embodiment, an array of oligonucleotides probes comprising ITGL-TSP nucleotide sequence or fragments thereof can be constructed to conduct efficient screening of e.g., genetic mutations. Array technology methods are well known and have general applicability and can be used to address a variety of questions in molecular genetics including gene expression, genetic linkage, and genetic variability. (See for example: M.Chee et al., Science, Vol 274, pp 610-613 (1996)).

The diagnostic assays offer a process for diagnosing or determining a susceptibility to, angiogenic diseases (cancer, cancer metastasis, chronic inflammatory disorders, rheumatoid arthritis, atherosclerosis, macular degeneration, diabetic retinopathy), restenosis, Alzheimer's disease and tissue remodeling through detection of mutation in the ITGL-TSP gene by the methods described.

In addition, angiogenic diseases (cancer, cancer metastasis, chronic inflammatory disorders, rheumatoid arthritis, atherosclerosis, macular degeneration, diabetic retinopathy), restenosis, Alzheimer's disease and tissue remodeling can be diagnosed by methods comprising determining from a sample derived from a subject an abnormally decreased or increased level of the ITGL-TSP polypeptide or ITGL-TSP mRNA. Decreased or increased expression can be measured at the RNA level using any of the methods well known in the art for the quantitation of polynucleotides, such as, for example, PCR, RT-PCR, RNase protection, Northern blotting and other hybridization methods. Assay techniques that can be used to determine levels of a protein, such as an ITGL-TSP polypeptide, in a sample derived from a host are well-known to those of skill in the art. Such assay methods include radioimmunoassays, competitive-binding assays, Western Blot analysis and ELISA assays.

### Chromosome Assays

The nucleotide sequences of the present invention are also valuable for chromosome identification. The sequence is specifically targeted to and can hybridize with a particular location on an individual human chromosome. The mapping of relevant sequences to chromosomes according to the present invention is an important first step in correlating those sequences with gene associated disease. Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes).

The differences in the cDNA or genomic sequence between affected and unaffected individuals can also be determined. If a mutation is observed in some or all of the affected individuals but not in any normal individuals, then the mutation is likely to be the causative agent of the disease. From our data we estimated that ITGL-TSP gene maps between STS markers D21S1435 and D21S1442 which translates as 21q21. This is a similar chromosomal location to amyloid precursor protein (APP), and thus, we have mapped APP in relation to ITGL-TSP. They are approximately 3 million bases apart which is not a massive distance in human genomics. The chromosomal location includes important genes such as enterokinases (enzymes that activate trypsinogen by converting it to trypsin) and genes responsible for Alzheimer's disease.
We mapped the ITGL-TSP to the 21q21 chromosomal location. The oligo sequences that were used for Radiation Hybrid mapping are as follows:

F 5' actgtgtgtgatccgag 3' (SEQ ID NO: 3)

R 5' gttggaaagcattgacg 3' (SEQ ID NO: 4)

Briefly, sequences can be mapped to chromosomes by preparing PCR primers (preferably 15-25 bp) from the cDNA. Computer analysis of the cDNA is used to rapidly select primers that do not span more than one exon in the genomic DNA, thus complicating the amplification process. These primers are then used for PCR screening of somatic cell hybrids containing individual human chromosomes. Only those hybrids containing the human gene corresponding to the primer will yield an amplified fragment.

PCR mapping of somatic cell hybrids is a rapid procedure for assigning a particular DNA to a particular chromosome. Using the present invention with the same oligonucleotide primers, sublocalization can be achieved with panels of fragments from specific chromosomes or pools of large genomic clones in an analogous manner. Other mapping strategies that can similarly be used to map to its chromosome include Radiation Hybrid mapping , prescreening with labeled flow-sorted chromosomes and preselection by hybridization to construct chromosome specific-cDNA libraries. Radiation Hybrid (RH) mapping relies upon fragmentation of human chromosomes with X-rays, and retention of these random fragments in Hamster A23 host cells. The DNAs for RH mapping are supplied by Research Genetics (USA). Oligo pairs are designed from EST sequences that will amplify products of between 80bp and 300bp. The PCRs are performed on 93 human/hamster hybrid DNAs and the results compared with a framework map (http://www-genome.wi.mit.edu/cgi-bin/contig/rhmapper.pl, Gyapay, G et al 1996) Human Molecular Genetics 5 : 339-346. RH mapping provides greater precision than FISH and indicates clusters of genes as well as disease locus/gene correlations.

### Anibodies

The polypeptides of the invention or their fragments or analogs thereof, or cells expressing them can also be used as immunogens to produce antibodies immunospecific for the ITGL-TSP polypeptides. The term "immunospecific" means that the antibodies have substantiall greater affinity for the polypeptides of the invention than their affinity for other related polypeptides in the prior art.

Antibodies generated against the ITGL-TSP polypeptides can be obtained by administering the polypeptides or epitope-bearing fragments, analogs or cells to an animal, preferably a nonhuman, using routine protocols. For preparation of monoclonal antibodies, any technique which provides antibodies produced by continuous cell line cultures can be used. Examples include the hybridoma technique (Kohler, G. and Milstein, C., *Nature* (1975) 256:495497), the trioma technique, the human B-cell hybridoma technique (Kozbor *et al., Immunology Today* (1983) 4:72) and the EBV-hybridoma technique (Cole *et al.,* MONOCLONAL ANTIBODIES AND CANCER THERAPY, pp. 77-96, Alan R. Liss, Inc., 1985).

Techniques for the production of single chain antibodies (U.S. Patent No. 4,946,778) can also be adapted to produce single chain antibodies to polypeptides of this invention. Also, transgenic mice, or other organisms including other mammals, may be used to express humanized antibodies.

The above-described antibodies may be employed to isolate or to identify clones expressing the polypeptide or to purify the polypeptides by affinity chromatography.

Antibodies against ITGL-TSP polypeptides may also be employed to angiogenic diseases (cancer, cancer metastasis, chronic inflammatory disorders, rheumatoid arthritis, atherosclerosis, macular degeneration, diabetic retinopathy), restenosis, Alzheimer's disease and tissue remodeling, among others.

### Vaccines

Another aspect of the invention relates to a method for inducing an immunological response in a mammal which comprises inoculating the mammal with ITGL-TSP polypeptide, or a fragment thereof, adequate to produce antibody and/or T cell immune response to protect said animal from , angiogenic diseases (cancer, cancer metastasis, chronic inflammatory disorders, rheumatoid arthritis, atherosclerosis, macular degeneration, diabetic retinopathy), restenosis, Alzheimer's disease and tissue remodeling, among others. Yet another aspect of the invention relates to a method of inducing immunological response in a mammal which comprises, delivering ITGL-TSP polypeptide via a vector directing expression of ITGL-TSP polynucleotide *in vivo* in order to induce such an immunological response to produce antibody to protect said animal from diseases.

Further aspect of the invention relates to an immunological/vaccine formulation (composition) which, when introduced into a mammalian host, induces an immunological response in that mammal to a ITGL-TSP polypeptide wherein the composition comprises a ITGL-TSP polypeptide or ITGL-TSP gene. The vaccine formulation may further comprise a suitable carrier. Since ITGL-TSP polypeptide may be broken down in the stomach, it is preferably administered parenterally (including subcutaneous, intramuscular, intravenous, intradermal etc. injection). Formulations suitable for parenteral administration include aqueous and non-aqueous sterile injection solutions which may contain anti-oxidants, buffers, bacteriostats and solutes which render the formulation instonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

### Screening Assays

The ITGL-TSP polypeptide of the present invention may be employed in a screening process for compounds which activate (agonists) or inhibit activation of (antagonists, or otherwise called inhibitors) the ITGL-TSP polypeptide of the present invention. Thus, polypeptides of the invention may also be used to assess identify agonist or antagonists from, for example, cells, cell-free preparations, chemical libraries, and natural product mixtures. These agonists or antagonists may be natural substrates, ligands, receptors, etc., as the case may be, of the polypeptide of the present invention; or may be structural or functional mimetics of the polypeptide of the present invention. See Coligan *et al., Current Protocols in Immunology* 1(2):Chapter 5 (1991).

ITGL-TSP polypeptides are responsible for many biological functions, including many pathologies. Accordingly, it is desirous to find compounds and drugs which stimulate ITGL-TSP polypeptide on the one hand and which can inhibit the function of ITGL-TSP polypeptide on the other hand. In general, agonists are employed for therapeutic and prophylactic purposes for such conditions as , angiogenic diseases (cancer, cancer metastasis, chronic inflammatory disorders, rheumatoid arthritis, atherosclerosis, macular degeneration, diabetic retinopathy), restenosis, Alzheimer's disease and tissue remodeling. Antagonists may be employed for a variety of therapeutic and prophylactic purposes for such conditions, angiogenic diseases (cancer, cancer metastasis, chronic inflammatory disorders, rheumatoid arthritis, atherosclerosis, macular degeneration, diabetic retinopathy), restenosis, Alzheimer's disease and tissue remodeling.

In general, such screening procedures may involve using appropriate cells which express the ITGL-TSP polypeptide or respond to ITGL-TSP polypeptide of the present invention. Such cells include cells from mammals, yeast, *Drosophila* or *E. coli.* Cells which express the ITGI-TSP polypeptide (or cell membrane containing the expressed polypeptide) or respond to ITGL-TSP polypeptide are then contacted with a test compound to observe binding, or stimulation or inhibition ot a functional response. The ability of the cells which were contacted with the candidate compounds is compared with the same cells which were not contacted for ITGL-TSP activity. The assays which will be routinely used are: Enzyme-linked immunosorbent sandwich assay (ELISA); receptor binding/inhibition assay; inhibition of cell adhesion/migration/proliferation assay; assays which utilize neutralizing mAbs against other integrin ligands/receptors; competition assays with integrin ligands/receptors.

The assays may simply test binding of a candidate compound wherein adherence to the cells bearing the ITGL-TSP polypeptide is detected by means of a label directly or indirectly associated with the candidate compound or in an assay involving competition with a labeled competitor. Further, these assays may test whether the candidate compound results in a signal generated by activation of the ITGL-TSP polypeptide, using detection systems appropriate to the cells bearing the ITGL-TSP polypeptide. Inhibitors of activation are generally assayed in the presence of a known agonist and the effect on activation by the agonist by the presence of the candidate compound is observed. Standard methods for conducting such screening assays are . well understood in the art.

Examples of potential ITGL-TSP polypeptide antagonists include antibodies or, in some cases, oligonucleotides or proteins which are closely related to the ligands, substrates, receptors, etc., as the case may be, of the ITGL-TSP polypeptide, e.g., a fragment of the ligands, substrates, receptors, or small molecules which bind to the polypetide of the present invention but do not elicit a response, so that the activity of the polypeptide is prevented.

### Prophylactic and Therapeutic Methods

This invention provides methods of treating an abnormal conditions related to both an excess of and insufficient amounts of ITGL-TSP polypeptide activity.

If the activity of ITGL-TSP polypeptide is in excess, several approaches are available. One approach comprises administering to a subject an inhibitor compound (antagonist) as hereinabove described along with a pharmaceutically acceptable carrier in an amount effective to inhibit activation by blocking binding of ligands to the ITGL-TSP polypeptide, or by inhibiting a second signal, and thereby alleviating the abnormal condition.

In another approach, soluble forms of ITGL-TSP polypeptides still capable of binding the ligand in competition with endogenous ITGL-TSP polypeptide may be administered. Typical embodiments of such competitors comprise fragments of the ITGL-TSP polypeptide.

In still another approach, expression of the gene encoding endogenous ITGL-TSP polypeptide can be inhibited using expression blocking techniques. Known such techniques involve the use of antisense sequences, either internally generated or separately administered. See, for example, O'Connor, *J Neurochem* (1991) 56:560 in Oligodeoxynucleotides as Antisense Inhibitors of Gene Expression, CRC Press, Boca Raton, FL (1988). Alternatively, oligonucleotides which form triple helices with the gene can be supplied. See, for example, Lee *et al., Nucleic Acids Res* (1979) 6:3073; Cooney *et al., Science* (1988) 241:456; Dervan *et al., Science* (1991) 251:1360. These oligomers can be administered *per se* or the relevant oligomers can be expressed *in vivo.*

For treating abnormal conditions related to an under-expression of ITGL-TSP and its activity, several approaches are also available. One approach comprises administering to a subject a therapeutically effective amount of a compound which activates ITGL-TSP polypeptide, i.e., an agonist as described above, in combination with a pharmaceutically acceptable carrier, to thereby alleviate the abnormal condition. Alternatively, gene therapy may be employed to effect the endogenous production of ITGL-TSP by the relevant cells in the subject. For example, a polynucleotide of the invention may be engineered for expression in a replication defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells *in vivo* and expression of the polypeptide *in vivo.* For overview of gene therapy, see Chapter 20, *Gene Therapy and other Molecular Genetic based Therapeutic Approaches*, (and references cited therein) in Human Molecular Genetics, T Strachan and A P Read, BIOS Scientific Publishers Ltd (1996).

### Formulation and Administration

Peptides, such as the soluble form of ITGL-TSP polypeptides, and agonists and antagonist peptides or small molecules, may be formulated in combination with a suitable pharmaceutical carrier. Such formulations comprise a therapeutically effective amount of the polypeptide or compound, and a pharmaceutically acceptable carrier or excipient. Such carriers include but are not limited to, saline, buffered saline, dextrose, water, glycerol, ethanol, and combinations thereof. Formulation should suit the mode of administration, and is well within the skill of the art. The invention further relates to pharmaceutical packs and kits comprising one or more containers filled with one or more of the ingredients of the aforementioned compositions of the invention.

Polypeptides and other compounds of the present invention may be employed alone or in conjunction with other compounds, such as therapeutic compounds.

Preferred forms of systemic administration of the pharmaceutical compositions include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if properly formulated in enteric or encapsulated formulations, oral administration may also be possible. Administration of these compounds may also be topical and/or localized, in the form of salves, pastes, gels and the like.

The dosage range required depends on the choice of peptide, the route of administration, the nature of the formulation, the nature of the subject's condition, and the judgment of the attending practitioner. Suitable dosages, however, are in the range of 0.1-100 µg/kg of subject. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as "gene therapy" as described above. Thus, for example, cells from a subject may be engineered with a polynucleotide, such as a DNA or RNA, to encode a polypeptide *ex vivo,* and for example, by the use of a retroviral plasmid vector. The cells are then introduced into the subject.

### Examples

The example below is carried out using standard techniques, which are well known and routine to those of skill in the art, except where otherwise described in detail. The example illustrates, but does not limit the invention.

### Example 1

### ITGL-TSP Cloning Strategy

### Cloning history:

The Metalloprotease with TSR homology clone (METH-1) was first identified ina cDNA library prepared from human adipocytes obtained from an osteoclastoma. The clone was identified as a novel human protein possessing homology to the thrombospondin type 1 repeat, as well as to several hemorrhagic proteins. The initial identification of the METH-1, cDNA clone was made using a previously described thrombospondin type 1 repeat sequence as a query sequencce in the BLASTN and TBLASTN sequence alignment algorithms (Altschul, S.F., Gish, W., Miller, W., Myers, E.W., and Lipman, D. J. (1990) *J. Mol. Biol.* **215**:403-410) against the Human Genome Sciences, Inc. EST database. A single EST clone was identified by this method as a potentially novel human hemorrhagic protein.

The cDNA clone initially identified in the BLAST analyses was thought to lack about 1.4 kb of 5' sequence, and, as a result, did not appear to be a full-length clone. However, the clone was useful as a probe to perform additional screens to obtain a full-length cDNA copy of the METH-1 gene. In this regard, METH-1-specific oligonucleotides were designed from sequence information obtained from the partial cDNA and were then used in conjunction with the Gene Trapper™ cDNA Positive Selection System kit (Life Technologies, Grand Island, NY) to screen a human pCMVSport kidney cDNA library for a full-length clone. Briefly, a biotinylated METH-1-specific oligonucleotide was hybridized to a complex population consisting of single-stranded copies of the 10⁶ to 10⁷ individual cDNA clones which make up the human pCMVSport kidney library. Hybrids consisting of the biotinylayed METH-1-specific oligonucleotide hybridized to various single-stranded cDNA clones were captured by streptavidin-coated magnetic beads. A magnet was used to separate the magnetic beads from the solution which contained the entire single-stranded library. Following several washing steps, the single-stranged cDNA clone was primed with Klenow DNA polymerase using a second METH-1-specific oligonucleotide. ElectroMAX DH10B™ electrocompetent E. coli cells (Life Technologies, Grand Island, NY) were transformed with the rescued cDNA clones and PCR was used to screen the resulting colonies for full-length cDNa clones of the METH-1 gene. The full-length cDNA copy of the METH-1 ORF was subsequently cloned into the bacterial and baculovirus expression vectors pQE-9 (Qiagen, Inc., Chatsworth, CA) and pA2GP, respectively, for the production and purification of METH-1 protein.

## Claims

1. An isolated polynucleotide comprising a nucleotide sequence that has at least 80% identity to a nucleotide sequence encoding the ITGL-TSP polypeptide of SEQ ID NO:2 over its entire length: or a nucleotide sequence complementary to said nucleotide sequence.

2. The polynucleotide of claim I which is DNA or RNA.

3. The polynucleotide of claim I wherein said nucleotide sequence is at least 80% identical to that contained in SEQ ID NO:1.

4. The polynucleotide of claim 3 wherein said nucleotide sequence comprises the ITGL-TSP polypeptide encoding sequence contained in SEQ ID NO:1.

5. The polynucleotide of claim 3 which is polynucleotide of SEQ ID NO: 1.

6. A DNA or RNA molecule comprising an expression system, wherein said expression system is capable of producing a ITGL-TSP polypeptide comprising an amino acid sequence, which has at least 80% identity with the polypeptide of SEQ ID NO:2 when said expression system is present in a compatible host cell.

7. A host cell comprising the expression system of claim 6.

8. A process for producing a ITGL-TSP polypeptide comprising culturing a host of claim 7 under conditions sufficient for the production of said polypeptide and recovering the polypeptide from the culture.

9. A process for producing a cell which produces a ITGL-TSP polypeptide thereof comprising transforming or transfecting a host cell with the expression system of claim 6 such that the host cell, under appropriate culture conditions. produces a ITGL-TSP polypeptide.

10. A ITGL-TSP polypeptide comprising an amino acid sequence which is at least 80% identical to the amino acid sequence of SEQ ID NO:2 over its entire length.

11. The polypeptide of claim 10 which comprises the amino acid sequence of SEQ ID NO:2.

12. An antibody immunospecific for the ITGL-TSP polypeptide of claim 10.

13. A method for the treatment of a subject in need of enhanced activity or expression of ITGL-TSP polypeptide of claim 10 comprising:
(a) administering to the subject a therapeutically effective amount of an agonist to said polypeptide; and/or
(b) providing to the subject a polynucleotide of claim 1 in a form so as to effect production of said polypeptide activity *in vivo*.

14. A method for the treatment of a subject having need to inhibit activity or expression of ITGL-TSP polypeptide of claim 10 comprising:
(a) administering to the subject a therapeutically effective amount of an antagonist to said polypeptide; and/or
(b) administering to the subject a nucleic acid molecule that inhibits the expression of the nucleotide sequence encoding said polypeptide; and/or
(c) administering to the subject a therapeutically effective amount of a polypeptide that competes with said polypeptide for its ligand, substrate, or receptor.

15. A process for diagnosing a disease or a susceptibility to a disease in a subject related to expression or activity of ITGL-TSP polypeptide of claim 10 in a subject comprising:
(a) determining the presence or absence of a mutation in the nucleotide sequence encoding said ITGL-TSP polypeptide in the genome of saia subject; and/or
(b) analyzing for the presence or amount of the ITGL-TSP polypeptide expression in a sample derived from said subject.

16. A method for identifying compounds which inhibit (antagonize) or agonize the ITGL-TSP polypeptide of claim 10 which comprises:
(a) contacting a candidate compound with cells which express the ITGL-TSP polypeptide (or cell membrane expressing ITGL-TSP polypeptide) or respond to ITGL-TSP polypeptide; and
(b) observing the binding, or stimulation or inhibition of a functional response; or comparing the ability of the cells (or cell membrane) which were contacted with the candidate compounds with the same cells which were not contacted for ITGL-TSP polypeptide activity.

17. An agonist identified by the method of claim 16.

18. An antagonist identified by the method of claim 16.
